# EUROPEAN PATENT SPECIFICATION

(11) **EP 2 318 086 B1**
(45) Date of publication and mention of the grant of the patent: **13.04.2016**
(21) Application number: 09786417.7
(22) Date of filing: 09.06.2009
(51) Int. Cl.: A61K 9/00, A61M 37/00, A61K 41/00, A61K 49/22

(54) **ULTRASOUND MEDIATED DRUG DELIVERY**
ULTRASCHALLVERMITTELTE WIRKSTOFFFREISETZUNG
ADMINISTRATION DE MÉDICAMENT INDUITE PAR LES ULTRASONS

(30) Priority: 23.07.2008 US 61690 P
(43) Date of publication of application: 11.05.2011
(73) Proprietor: Koninklijke Philips N.V., 5656 AE Eindhoven (NL)
(72) Inventor: SHI, William, Bothell Washington 98041-3003 (US); CHIN, Chien, Ting, Briarcliff Manor New York 10510-8001 (US); HALL, Christopher, Bothell Washington 98041-3003 (US); RAJU, Balasundara, Bothell Washington 98041-3003 (US); BOHMER, Marcel, Briarcliff Manor New York 10510-8001 (US)
(74) Representative: van Velzen, Maaike Mathilde
(86) International application number: PCT/IB2009/052449
(87) International publication number: WO 2010/010473

(56) References cited:
- WO-A-2006/131840
- WO-A1-2005/070473
- US-A- 5 618 275
- US-A- 6 135 971
- US-B1- 6 322 532

## Description

### FIELD OF THE INVENTION

The invention relates to the administration and delivery of Therapeutic Agents mediated by the application of ultrasound.

### BACKGROUND OF THE INVENTION

Therapeutic use of ultrasound-activated drug-bearing particles is an emerging technique with potential for a variety of clinical applications. In the area of ultrasound contrast imaging and drug delivery, both regular (non-targeted) and targeted contrast agents may be utilized. For non-site specific contrast agents, targeted drug delivery can be achieved by accurate focusing of ultrasound energy at the region of interest. Ultrasound contrast agents, typically between 0.1 and 8 µm in diameter, increase the echo amplitude from the blood pool and allow the detection of vessels even as small as capillaries.

The rationale for using ultrasound mediated drug delivery is based on the need for site specific applications, such as organ or tissue-specific, tumor-specific or clot-specific pharmacotherapy. Drugs can be incorporated into particles capable of ultrasound activation (microbubbles, nanoparticles, and liposomes) by a variety of different approaches. Targeted drug delivery with this technology is probably most useful for highly active drugs that do not require large payloads of drug for biological effect. Many chemotherapeutics, proteins, gene based drugs, thrombolytics, and other therapeutic agents are sufficiently active for delivery with ultrasound and targeted acoustically active carriers. With the development of new targeting and drug release planning strategies, ultrasonically active particles may become an important therapeutic tool for targeted treatments.

Cancers and cardiovascular diseases together account for over 57% of all deaths in the US. These diseases are usually localized and are often alleviated by focal treatments such as surgery and radiation therapy. Many drugs and radiation have negative side effects on normal tissue which results in reduced survival rates and quality of life. The side effects of many existing drugs can be reduced by localizing their effects to the disease site. On the other hand, emerging forms of therapies employing genetic technologies are often limited not by side effects but by the low delivery efficiency of the genetic materials to the interior of the target cells.

Many biochemical techniques are being developed to increase localization and to enhance uptake of the therapeutic agent. Targeting molecules, such as monoclonal antibodies, promise to provide excellent specificity. Other microscopic constructions are designed to package therapeutic agents and release them locally under certain conditions, e.g., the detection of a disease-specific molecular species. However, such approaches remain unpractical due in large part to the nearly intractable complexity of interacting biological processes.

Ultrasound offers an alternative to complex biological process for controlling the release or activation of the therapeutic agents. Certain carrier vehicle of drugs, such as microbubbles and nano-droplets, can be triggered by suitable ultrasound to release their contents. In case of gene delivery, where the low efficacy is more problematic than side effects, it may not even be necessary to package the therapeutic agents into the microbubbles. Simply mixing the bubbles with the agent might be sufficient to achieve increased efficacy. The most likely mechanism for such effects is that as the bubbles oscillate in the ultrasound field, microscopic fluid motions drive the therapeutic agents into cells or temporarily open pores in the cell membrane allowing free movement of molecules. It is also known that ultrasound in the presence of microbubbles can open the blood-brain barrier, allowing drugs to reach the target cells in the brain (Hynynen, Harvard Medical School). WO 2005/07043 describes an ultrasound contrast agent which comprises metal nano-particles. The metal nanoparticles are stable, biocompatible and can be coupled to bio-target-specific molecules for targeted visualization. The particles can also be used for drug delivery.

All therapeutic drugs are given within an intended dosage range. Below this range, the desired therapeutic effect would not be achieved; above this range, undesired toxic effects can develop. Microbubble mediated drug delivery technologies are generally intended to improve control over the delivery process. However, there is as yet no technology that measures the actual dosage level delivered to the target site, either as a static measurement after the treatment or as continuous or repeated measurements during the treatment. Without such information, future clinicians will have to perform these novel medical procedure "blindly". Ultrasound is typically used to release the therapeutic agent or cause the therapeutic agent to be taken up by the target tissue. A number of factors influence the accurate delivery of the ultrasound energy so that the desired effect is triggered in the diseased region. The diseased region is typically in motion due to internal organ motions (such as the heartbeat) or gross patient motion. Fat, bones, air and fluid-filled regions in the patient body also modify or degrade the ultrasound beam before it reaches the target site. Therefore, real-time or repeated adjustments to the ultrasound firing device are necessary to achieve optimal treatment.

A reference on ultrasound-mediated drug delivery is US 2003/0204141. Herein a method and system are described for both enhancing drug uptake by application of therapeutic transmissions of acoustic energy and imaging a region to help guide the therapy with a same transducer and ultrasound system. As to the drug delivery part of the method, this reference addresses nothing less and nothing more than the ultrasound-mediation *per se.* The imaging too is known imaging of e.g. a target tissue.

WO 2006/131840 describes a method and apparatus for ultrasound drug delivery and thermal therapy with phase-convertible fluids. The ultrasound treatment comprises applying ultrasound at a first energy level to a region of interest in a subject for activating a caviation nucleation agent during a first portion of a treatment cycle. Ultrasound at a second energy level is applied to the region of interest during a second portion of the treatment cycle for implementing a desired thermal therapy in the presence of the cavitation nucleation agent activated during the first portion of the treatment cycle.

### SUMMARY OF THE INVENTION

It would be advantageous to provide a method of ultrasound-mediated delivery of therapeutic agents that would allow a better setting of the dosage level of the agent. It would further be advantageous to provide a method of ultrasound-mediated delivery of therapeutic agents that would allow a more accurate spatial control over drug delivery. Particularly, it would be desired to provide a method of ultrasound-mediated delivery of therapeutic agents that would allow an enhanced targeting accuracy and drug release efficiency.
In order to better address at least one of the foregoing desires, the invention, in one aspect, presents a composition for use in a method according to claim 1

The detection of the acoustic properties herein allows using the information obtained on the change of the acoustic properties of the Particles of the second population in tuning one or more parameters relevant to the delivery of the Therapeutic Agent by means of particles of the first population.

It should be noted that the detection of acoustic properties refers to a physical step in which, as a technical measure, particles before, during or after subjection to ultrasound are detected by a detector (generally an ultrasound imaging system). The information received from this detection is interpreted and further used, generally by means of a suitable software-supported method, using a computer as a technical means.

The administration of Ultrasound Particles and of Ultrasound Dosage Forms typically is by intravenous administration, although other types of administration are not excluded (e.g. application into a body cavity or intra-arterial or intra-lymphatic system).

### DETAILED DESCRIPTION OF THE INVENTION

In a broad sense, the invention can be described with reference to any method in which the release of a Therapeutic Agent is influenced by the application of ultrasonic energy (ultrasound). According to the invention, in such a method a novel step is included in which information is obtained on relevant behavior of administered particles that care capable of activation by ultrasound, and the information so obtained is used in adapting the behavior of ultrasound-mediated particles releasing a Therapeutic Agent.

The change in acoustic properties, e.g. in the form of echoes and/or emission of nonlinear ultrasonic signals is detected and quantified by an ultrasonic detector in either active pulse-echo mode or passive receive-only mode. The detected signal or signal change is converted, in conjunction with *a priori* knowledge of the quantity of the therapeutic agent per quantity unit of the carrier, into an estimate of a delivered dose level of agent. Multiple independent estimates of the dose level can be collected and accumulated over time and from different regions of the treated and neighboring organs to build up a complete, dynamic picture of the progress of the treatment.

The treatment system can display the dosage information in a human-understandable format and the clinician or operator can use the information to adapt, optimize or terminate the treatment procedure. Alternatively, the treatment system can employ an algorithm that controls the agent release process such that an optimal dose is delivered to a region ideally matched to the diseased organ.

For a proper understanding of the inventive concept disclosed herein with reference to the detection of acoustic properties, an explanation is first given of various terms used in describing this invention. The mammal, and particularly a human person, to which the invention can be applied, is hereinafter referred to as 'person.'

### Ultrasound

Ultrasound is well-known in the art as acoustic energy that can be applied for imaging (e.g. fetuses in the womb). Increasingly, ultrasound is also described as a source of external energy that can affect drug release, by affecting physical properties of ultrasoundsensitive carriers.

Ultrasound is generally applied by means of a transducer probe that sends (and receives) ultrasonic sound waves. When using ultrasound to activate drug delivery, the basic requirement is that ultrasonic waves can be transmitted into a person's body (i.e. the receiving is of secondary importance here). In carrying out the invention, a therapy applicator is thus provided that serves to transmit ultrasonic waves into a person's body. Such applicators are known, as described e.g. in WO 2006/131840.

As a general advantage in the therapeutic application of ultrasound, it will be preferred to employ equipment that not only serves to transmit the ultrasound into the body, but that also enables imaging of body tissue and the like (e.g. a tumor or a blood clot). To this end the equipment as described in the aforementioned US 2003/0204141 can be used, or any other ultrasound imaging equipment. Such equipment is known in the art, as described in e.g. US 2007/0255117, US 6,527,718, US 7,358,226, WO 2006/131840.

### Ultrasound Particles

This generally refers to particles such as microbubbles, microparticles, nanoparticles, microcapsules or nanocapsules, having in common that they undergo a physical change upon the application of ultrasound. This change can effect e.g. the particles' physical state (e.g. they can melt) or integrity (e.g. ultrasound-mediated destruction of microbubbles) or shape/size (e.g. they can oscillate in size) or porosity (e.g. they can make their payload temporarily available to the surrounding medium).

Particles capable of activation by ultrasound (hereinafter referred to as Ultrasound Particles) include aqueous suspensions of gaseous microbubbles. These exhibit desirably large differences in acoustic impedance between a gas (e.g., air) and the surrounding aqueous medium. Such microbubbles are capable of enhancing ultrasound signals by a factor of up to a few hundreds. Detailed descriptions of the development of ultrasound contrast agents are given in the reviews by C. J. Harvey et al., Eur. Radiol. vol. 11, pp. 675-689 (2001), and J-M. Correas etal., Eur. Radiol. Vol. 11, pp.1316-1328 (2001). A size requirement for these microbubbles exists; they must be injectable intravenously and be small enough to pass through the capillaries of most tissues; i.e. , they have to be smaller than about 8 microns. However, very small microbubbles (ca. 1 micron) are not very echogenic, and a compromise has to be made. To posses high echogenicity but still pass through the capillaries of most tissues, 3-4 microns are considered to be an optimal size (A. L.Klibanov,"Ultrasound Contrast Agents : Development of the field and current status"in Topics in Current Chemistry, Vol. 222, p. 73, Springer- Verlag Berlin, Heidelberg (2002)). In addition, the size of the particle controls the optimal imaging frequency or resonance of the particle, and therefore 2 to 4 micron diameter particles are preferably chosen because the resonance lies in the medical diagnostic imaging frequency range of 1 to 10 MHz.

An early disclosure of drug delivery using ultrasound imaging contrast agents for drug delivery is WO 97/33474. Herein it is explained how microbubbles will cavitate under the influence of ultrasound, as a result of which a conjugated bioactive agent will be released and can thus be delivered to a target site. In addition to the microbubbles described in WO 97/33474, many other suitable particles are disclosed in the art. Reference is made to, e.g., WO 2005/039750, which describes core-shell microparticles made by mixing a polyelectrolyte microgel and a colloid in an aqueous solution. US patents 5,487,390 and 5,562,099 describe respectively gas-filled and contrast agent-filled polymeric microcapsules for ultrasound imaging, formed by ionotropically gelling synthetic polyelectrolytes by contact with multivalent ions. WO 89/06978 describes ultrasonic contrast agents consisting of micro- particles containing amyloses or synthetic biodegradable polymers. EP 0441468 describes ultrasound contrast agents consisting of microparticles having a particle diameter of from 0.1 to 40 microns consisting of a biodegradable polymer obtainable from a polymerisable aldehyde and a gas and/or liquid having a boiling point of less than 60°C. EP 0576519 describes ultrasound contrast agents comprising gas-filled vesicles described as "microballoons" comprising microbubbles of gas encapsulated by monolayers or one or more bilayers of non-proteinaceous crosslinked or polymerised amphiphilic moieties. US 2002/0150539 and US 2005/0123482 describe gaseous precursor-filled liposomes suitable for use as contrast agents for ultrasonic imaging or as drug delivery agents. WO 00/72757 describes surface stabilized microbubbles for use in ultrasound contrast and drug delivery agents. Polymeric particles, partially filled with a gas or a gas-precursor, for use in ultrasound-mediated drug delivery are described in WO 2007/010442. In US 2006/0002994 liposomes with enhanced ultrasound responsiveness are described, suitable for ultrasound-mediated drug delivery, that are based on the incorporation of surface active dopants containing ethylene glycol polymers or oligomers.

### Ultrasound Dosage Form

This refers to essentially the same types of Ultrasound Particles described hereinbefore, with the added feature of comprising a Therapeutic Agent. The Therapeutic Agent can be bound to the Ultrasound Particles, e.g. by chemical interaction (e.g. covalently) or by physical interaction (e.g. adsorption). The Therapeutic Agent can also be incorporated into a cavity of the Ultrasound Particles. It is also conceivable that the Ultrasound Particles comprise the Therapeutic Agent in such a way as to be merely mixed together.

### Therapeutic Agent

This refers to any bioactive agent that is useful to be administered through ultrasound mediation. This includes truly therapeutic agents, that serve the treatment of a disease or disorder, as well as agents for prophylaxis that serve to prevent the occurrence, or worsening, of a disease or disorder. It also includes genetic material such as plasmid DNA or siRNA.

Accordingly, compounds envisaged for use as bioactive agents in the context of the present invention include any compound with therapeutic or prophylactic effects. It can be a compound that affects or participates in tissue growth, cell growth, cell differentiation, a compound that is able to invoke a biological action such as an immune response, or a compound that can play any other role in one or more biological processes. A non-limiting list of examples includes antimicrobial agents (including antibacterial, antiviral agents and anti-fungal agents), anti-viral agents, anti-tumor agents, thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, anti metabolites, antiproliferatives (including antiangiogenesis agents), anticancer chemotherapeutic agents, anti-inflammatory steroid or non-steroidal anti-inflammatory agents, immunosuppressive agents, growth hormone antagonists, growth factors, dopamine agonists, radiotherapeutic agents, extracellular matrix components, ACE inhibitors, free radical scavengers, chelators, antioxidants, anti polymerases, and photodynamic therapy agents.

Relatively small peptides may be referred to by the number of amino acids (e.g. di-, tri-, tetrapeptides). A peptide with a relatively small number of amide bonds may also be called an oligopeptide (up to 50 amino acids), whereas a peptide with a relatively high number (more than 50 amino acids) may be called a polypeptide or protein. In addition to being a polymer of amino acid residues, certain proteins may further be characterized by the so-called quaternary structure, a conglomerate of a number of polypeptides that are not necessarily chemically linked by amide bonds but are bonded by forces generally known to the skilled professional, such as electrostatic forces and Vanderwaals forces. The term peptides, proteins or mixtures thereof as used herein is to include all above mentioned possibilities.

Usually, the protein and/or peptide are selected on the basis of its biological activity. Depending on the type of polymer chosen, the product obtainable by the present process is highly suitable for controlled release of proteins and peptides. In a particular embodiment, the protein or peptide is a growth factor.

Other examples of peptides or proteins or entities comprising peptides or proteins which may advantageously be contained in the loaded polymer include, but are not limited to, immunogenic peptides or immunogenic proteins, which include, but are not limited to, the following:
Toxins such as diphtheria toxin and tetanus toxin.

Viral surface antigens or parts of viruses such as adenoviruses, Epstein-Barr Virus, Hepatitis A Virus, Hepatitis B Virus, Herpes viruses, HIV-1, HIV-2, HTLV-III, Influenza viruses, Japanese encephalitis virus, Measles virus, Papilloma viruses, Paramyxoviruses, Polio Virus, Rabies, Virus, Rubella Virus, Vaccinia (Smallpox) viruses and Yellow Fever Virus.

Bacterial surface antigens or parts of bacteria such as *Bordetella pertussis, Helicobacter pylori, Clostridium tetani, Corynebacterium diphtheria, Escherichia coli, Haemophilus influenza, Klebsiella* species, *Legionella pneumophila, Mycobacterium bovis, Mycobacterium leprae*, *Mycrobacterium tuberculosis, Neisseria gonorrhoeae, Neisseria meningitidis, Proteus species, Pseudomonas aeruginosa, Salmonella species, Shigella species, Staphylococcus aureus*, *Streptococcus pyogenes, Vibrio cholera* and *Yersinia pestis.*

Surface antigens of parasites causing disease or portions of parasites such as *Plasmodium vivax* (malaria), *Plasmodium falciparum* (malaria), *Plasmodium ovale* (malaria), *Plasmodium malariae* (malaria), *Leishmania tropica* (leishmaniasis), *Leishmania donovani),* leishmaniasis), *Leishmania branziliensis* (leishmaniasis), *Trypanosoma rhodescense* (sleeping sickness), *Trypanosoma gambiense* (sleeping sickness), *Trypanosoma cruzi* (Chagas' disease), *Schistosoma mansoni* (schistosomiasis), *Schistosomoma haematobium* (schistomiasis), *Schistosoma japonicum* (shichtomiasis), Trichinella spiralis (trichinosis), *Stronglyloides duodenale* (hookworm), *Ancyclostoma duodenale* (hookworm), *Necator americanus* (hookworm), *Wucheria bancrofti* (filariasis), *Brugia malaya* (filariasis), *Loa loa* (filariasis), *Dipetalonema perstaris* (filariasis), *Dracuncula medinensis* (filariasis), and *Onchocerca volvulus* (filariasis).

Immunoglobulins such as IgG, IgA, IgM, Antirabies immunoglobulin, and Antivaccinia immunoglobulin.

Antitoxin such as Botulinum antitoxin, diphtheria antitoxin, gas gangrene antitoxin, tetanus antitoxin.

Antigens which elicit an immune response against foot and mouth disease.

Hormones and growth factors such as follicle stimulating hormone, prolactin, angiogenin, epidermal growth factor, calcitonin, erythropoietin, thyrotropic releasing hormone, insulin, growth hormones, insulin-like growth factors 1 and 2, skeletal growth factor, human chorionic gonadotropin, luteinizing hormone, nerve growth factor, adrenocorticotropic hormone (ACTH), luteinizing hormone releasing hormone (LHRH), parathyroid hormone (PTH), thyrotropin releasing hormone (TRH), vasopressin, cholecystokinin, and corticotropin releasing hormone; cytokines, such as interferons, interleukins, colony stimulating factors, and tumor necrosis factors: fibrinolytic enzymes, such as urokinase, kidney plasminogen activator; and clotting factors, such as Protein C, Factor VIII, Factor IX, Factor VII and Antithrombin III.

Examples of other proteins or peptides are albumin, atrial natriuretic factor, renin, superoxide dismutase, alpha 1 -antitrypsin, lung surfactant proteins, bacitracin, bestatin, cydosporine, delta sleep-inducing peptide (DSIP), endorphins, glucagon, gramicidin, melanocyte inhibiting factors, neurotensin, oxytocin, somostatin, terprotide, serum thymide factor, thymosin, DDAVP, dermorphin, Met-enkephalin, peptidoglycan, satietin, thymopentin, fibrin degradation product, des-enkephalin- alpha -endorphin, gonadotropin releasing hormone, leuprolide, alpha -MSH and metkephamid.

Anti-tumor agents such as altretamin, fluorouracil, amsacrin, hydroxycarbamide, asparaginase, ifosfamid, bleomycin, lomustin, busulfan, melphalan, chlorambucil, mercaptopurin, chlormethin, methotrexate, cisplatin, mitomycin, cyclophosphamide, procarbazin, cytarabin, teniposid, dacarbazin, thiotepa, dactinomycin, tioguanin, daunorubicin, treosulphan, doxorubicin, tiophosphamide, estramucin, vinblastine, etoglucide, vincristine, etoposid, vindesin and paclitaxel.

### Antimicrobial agents comprising:

Antibiotics such as ampicillin, nafcillin, amoxicillin, oxacillin, azlocillin, penicillin G, carbenicillin, penicillin V, dicloxacillin, phenethicillin, floxacillin, piperacillin, mecillinam, sulbenicillin, methicillin, ticarcillin, mezlocillin , Cephalosporins: cefaclor, cephalothin, cefadroxil, cephapirin, cefamandole, cephradine, cefatrizine, cefsulodine, cefazolin, ceftazidim, ceforanide, ceftriaxon, cefoxitin, cefuroxime, cephacetrile, latamoxef, and cephalexin. Aminoglycosides such as amikacin, neomycin, dibekacyn, kanamycin, gentamycin, netilmycin, tobramycin. Macrolides such as amphotericin B, novobiocin, bacitracin, nystatin, clindamycin, polymyxins, colistin, rovamycin, erythromycin, spectinomycin, lincomycin, vancomycin Tetracyclines such as chlortetracycline, oxytetracycline, demeclocycline, rolitetracycline, doxycycline, tetracycline and minocycline. Other antibiotics such as chloramphenicol, rifamycin, rifampicin and thiamphenicol.

Chemotherapeutic agents such as the sulfonamides sulfadiazine, sulfamethizol, sulfadimethoxin, sulfamethoxazole, sulfadimidin, sulfamethoxypyridazine, sulfafurazole, sulfaphenazol, sulfalene, sulfisomidin, sulfamerazine, sulfisoxazole and trimethoprim with sulfamethoxazole or sulfametrole.

Urinary tract antiseptics such as methanamine, quinolones(norfloxacin, cinoxacin), nalidixic acid, nitro-compounds (nitrofurantoine, nifurtoinol) and oxolinic acid.

Drug for anaerobic infections such as metronidazole.

Drugs for tuberculosis such as aminosalicyclic acid, isoniazide, cycloserine, rifampicine, ethambutol, tiocarlide, ethionamide and viomycin.

Drugs for leprosy such as amithiozone, rifampicine, clofazimine, sodium sulfoxone and diaminodiphenylsulfone (DDS, dapsone).

Antifungal agents such as amphotericin B, ketoconazole, clotrimazole, miconazole, econazole, natamycin, flucytosine, nystatine and griseofulvin.

Antiviral agents such as aciclovir, idoxuridine, amantidine, methisazone, cytarabine, vidarabine and ganciclovir.

Chemotherapy of amebiasis such as chloroquine, iodoquinol, clioquinol, metronidazole, dehydroemetine, paromomycin, diloxanide, furoatetinidazole and emetine.

Anti-malarial agents such as chloroquine, pyrimethamine, hydroxychloroquine, quinine, mefloquine, sulfadoxine/pyrimethamine, pentamidine, sodium suramin, primaquine, trimethoprim and proguanil.

Anti-helminthiasis agents such as antimony potassium tartrate, niridazole, antimony sodium dimercaptosuccinate, oxamniquine, bephenium, piperazine, dichlorophen, praziquantel, diethylcarbamazine, pyrantel parmoate, hycanthone, pyrivium pamoate, levamisole, stibophen, mebendazole, tetramisole, metrifonate, thiobendazole and niclosamide.

Anti-inflammatory agents such as acetylsalicyclic acid, mefenamic acid, aclofenac, naproxen, azopropanone, niflumic acid, benzydamine, oxyphenbutazone, diclofenac, piroxicam, fenoprofen, pirprofen, flurbiprofen, sodium salicyclate, ibuprofensulindac, indomethacin, tiaprofenic acid, ketoprofen and tolmetin.

Anti-gout agents such as colchicine and allopurinol.

Centrally acting (opoid) analgesics such as alfentanil, methadone, bezitramide, morphine, buprenorfine, nicomorphine, butorfanol, pentazocine, codeine, pethidine, dextromoramide, piritranide, dextropropoxyphene, sufentanil and fentanyl.

Local anesthetics such as articaine, mepivacaine, bupivacaine, prilocaine, etidocaine, procaine, lidocaine and tetracaine.

Drugs for Parkinson's disease such as amantidine, diphenhydramine, apomorphine, ethopropazine, benztropine mesylate, lergotril, biperiden, levodopa, bromocriptine, lisuride, carbidopa, metixen, chlorphenoxamine, orphenadrine, cycrimine, procyclidine, dexetimide and trihexyphenidyl.

Centrally active muscle relaxants such as baclofen, carisoprodol, chlormezanone, chlorzoxazone, cyclobenzaprine, dantrolene, diazepam, febarbamate, mefenoxalone, mephenesin, metoxalone, methocarbamol and tolperisone. Corticosteroids comprising:
Mineralocorticosteroids such as cortisol, desoxycorticosterone and flurohydro cortisone.

Glucocorticosteroids such as beclomethasone, betamethasone, cortisone, dexamethasone, fluocinolone, fluocinonide, fluocortolone, fluorometholone, fluprednisolone, flurandrenolide, halcinonide, hydrocortisone, medrysone, methylprednisolone, paramethasone, prednisolone, prednisone and triamcinolone (acetonide). Androgens comprising:
Androgenic steroids used in therapy such as danazole, fluoxymesterone, mesterolone, methyltestosterone, testosterone and salts thereof.

Anabolic steroids used in therapy such as calusterone, nandrolone and salts thereof, dromostanolone, oxandrolone, ethylestrenol, oxymetholone, methandriol, stanozolol methandrostenolone and testolactone.

Antiandrogens such as cyproterone acetate.

Estrogens comprising estrogenic steroids used in therapy such as diethylstilbestrol, estradiol, estriol, ethinylestradiol, mestranol and quinestrol.

Anti-estrogens such as chlorotrianisene, clomiphene, ethamoxytriphetol, nafoxidine and tamoxifen.

Progestins such as allylestrenol, desogestrel, dimethisterone, dydrogesterone, ethinylestrenol, ethisterone, ethynadiol diacetate, etynodiol, hydroxyprogesterone, levonorgestrel, lynestrenol, medroxyprogesterone, megestrol acetate, norethindrone, norethisterone, norethynodrel, norgestrel, and progesterone.

### Thyroid drugs comprising:

Thyroid drugs used in therapy such as levothyronine and liothyronine

Anti-thyroid drugs used in therapy such as carbimazole, methimazole, methylthiouracil and propylthiouracil.

Apart from bioactive agents which are water soluble, other water-soluble compounds can be incorporated such as anti-oxidants, ions, chelating agents, dyes, imaging compounds.

Preferred therapeutic agents are in the area of cancer (e.g. antitumor) and cardiovascular disease (e.g. thrombus treatment or prevention). These constitute the best candidates among Therapeutic Agents that allow benefit from ultrasound-mediated delivery.

### Detection of Acoustic Properties

In the present invention a step is included that addresses, *inter alia,* the desire to gain a better insight, before, during or after treatment, into the appropriate setting of the dosage level of the Therapeutic Agent and the site and characteristics of the ultrasound applied to release the Therapeutic Agent.

In general, it is considered that if in one part of the process of ultrasound-mediated release of a Therapeutic Agent, acoustic properties are detected of administered Ultrasound Particles (on or after the point in time at which these are subjected to ultrasound), the information obtained therefrom can be applied to adapt or adjust the release of Therapeutic Agent in one or more subsequent parts of the process.

A visual display of the recently released as well as the accumulated dose can be presented to the operator. The dose information can be superimposed on an anatomic image, which can be either 2-dimensional or 3-dimensional, so that localization of delivered therapy can be registered to the patient's anatomy.

The control unit can come in various forms. It can be a separate or integrated hardware device, but it can also be a software component running on the scanner.

As a further hardware option, the applicator (that serves to send ultrasound waves into the person subjected to the administration of the particles) can be constructed as an add-on component to the scanner. If desired, the applicator can in fact be one and the same as the scanner.

The acoustic analysis and method of processing the information obtained, can be done in a variety of ways. By way of exemplary description, it is envisaged that the operator uses the delivered dose information to decide the optimal course of action. His or her options include: continuing the treatment, spatially shifting the applicator or its ultrasound beam, either in response to suboptimal placement of the treatment location or to create a new treatment spot, and terminating the treatment. As an option, the control unit can include functionalities to activate, deactivate and control various parameters on the applicator. A closed feedback control loop can then be implemented such that the actual delivered dose is optimally matched to a treatment plan pre-defined by the operator (e.g., a physician). As another option, information can be extracted from pulse-echo measurements, with signal processing, that enable adaptive control of ultrasound-mediated delivery. For example, acoustic attenuation and aberration can be estimated from pulse-echo data obtained as part of the image acquisition by the ultrasound scanner. Such information can be used to control the applicator adaptively for optimal agent delivery efficacy and minimal side effects.

The control unit can be fed with information on boundaries. E.g. it can be provided with an upper value (e.g. side effect bound) for delivered dosage to the operator in order to limit side effects. The control unit can similarly provide a lower (treatment) bound value for delivered dosage to the operator in order to guarantee sufficient efficacy. As a further option, an additional injection of an echogenic contrast agent can be administered for the purpose of treatment verification. For example, a contrast agent targeted to (expected) immune or other biological response to the therapy may be injected to reveal the accuracy and quality of the treatment session.

In one embodiment, at least two sets of Ultrasound Particles are administered and subjected to ultrasound, one (second) set being subjected to detection of acoustic properties, the other (first) set comprising and releasing the therapeutic agent. The second set of Ultrasound Particles thus in fact serves as an imaging tracer, the other set serves as delivery vehicles comprising and releasing the Therapeutic Agent.

Preferably, this embodiment is conducted as a two-phase process, comprising as a first phase a planning phase (in which the imaging tracer is administered and subjected to detection of acoustic properties, and as a second phase a treatment phase in which drug-bearing acoustically active particles (typically Ultrasound Dosage Forms) are administered, and subjected to release of the Therapeutic Agent.

It should be noted that the term "phase" does not necessarily refer to a single administration. In fact, in the planning phase, both the tracer distribution and therapeutic ultrasound beam placement for the determination of actual treatment zone are best mapped out with accurately one or more injections of the imaging tracer into bloodstream.

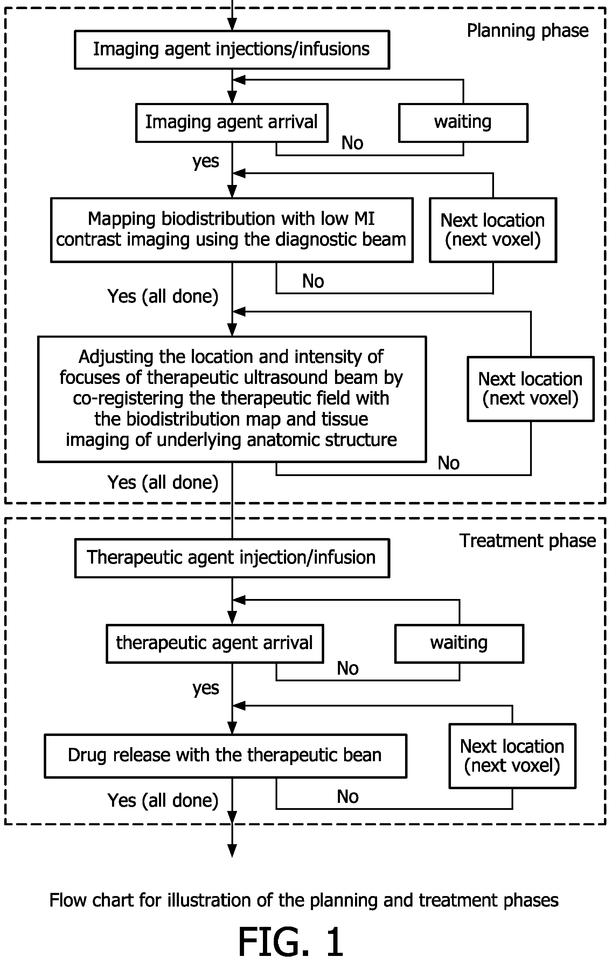

The invention thus describes a new approach for use of one or more acoustic tracers for ultrasound-assisted drug delivery. An exemplary scheme is given in Fig. 1. Herein, at least two known ultrasound agents are injected (or infused) one after another into bloodstream. The first one is a given number of stable, flexible microbubbles (e.g., lipid-shelled, perfluorocarbon gas-filled microbubbles) that acts as imaging tracers for continuous contrast imaging. This tracer provides information on the distribution of a subsequently administered drug and will also serve to map out and adjust the subsequent therapy beams. The second agent will act as the drug-delivery vehicle (Ultrasound Dosage Form) and contains a planned population of microcapsules, nanoparticles or liposomes or other acoustically active particles (Ultrasound Particles) as drug carriers for accurately targeted drug delivery. A combination of multiple tracers and/or multiple drug carriers can also be employed.

Generally, in the planning phase a relatively small known amount (calibrated before injection) of the first contrast agent injection is utilized to estimate the amount of drug required in the region of interest. This first step provides information on the quantity, concentration and contrast kinetics (such as wash-in, wash-out, as well as fractional blood volume) of contrast agent microbubbles in the region of interest. The first step is usually achieved using (not limited to) a low amplitude (i.e., low-MI) diagnostic ultrasound field. MI (Mechanical Index) is a measure for the likehood of ultrasound-induced mechanical bioeffects (particularly, harmful inertial cavitation). With the same injection (infusion) or after a new contrast injection, the therapeutic ultrasound field of the same imaging equipment or a new therapeutic device is turned on so that the focus of each therapeutic ultrasound beam can be visualized with limited microbubble destruction or with acoustic radiation force induced contrast microbubble displacement. In this way, the intensity of therapeutic ultrasound foci within the region of interest can be adjusted in comparison to the low-MI diagnostic ultrasound field. Both the distribution of the contrast agent (as predicted distribution of drug-bearing particles) and the actual focal locations of the therapeutic ultrasound field can be correlated and registered to ultrasound images (e.g., regular or harmonic B-mode and even Doppler imaging for large vessel landmarks) of the underlying anatomic structure.

Immediately at the end of the contrast injections or after a short period of time, a relatively large calibrated amount of drug-bearing particles flowing to the same region of interest may be activated according to the estimated local concentration and rate using information from the previous low-MI contrast imaging with the contrast agent population. Once the drug-bearing ultrasonically-active particles are detected or after a calibrated time period between the injection site and the region of interest is passed, the therapeutic beam is activated and scanned across the targeting region under the guidance of anatomic ultrasound imaging with registered maps for both contrast distribution and ultrasound focus location. The acoustic activation of drug-bearing agents is to disintegrate the agents in order to release their pharmaceutical payload through the use of ultrasound (e.g. at a higher MI and/or a greater pulse length). The activation can be initiated manually by the treating clinician or automatically using a programmable trigger.

The total amount, concentration and injection rate of the drug-carrying particles are calibrated and planned before injection. The ratio between the imaging microbubbles and the drug delivery vehicles is chosen such that sufficient drug can be incorporated, therefore the ratio can be 1:10 or even 1:100 or more. A combination of multiple carriers with multiple interactive drugs may be employed for optimal therapeutic effects.

This approach has the advantage that the imaging tracers can be interrogated repeatedly (either with low-MI contrast imaging or with multiple contrast injections) and its specific signature can be detected. In the case of targeted contrast agents, both the imaging bubble and the drug delivery vehicle can be modified with the same targeting moiety. The targeting accuracy may be greatly improved with a combination of targeted contrast agents and well focused ultrasound.

The minimum and maximum ultrasound energy levels (including center frequency, amplitude, pulse length as well as pulse repetition, frequency, or frame rate) required for non-destructive contrast imaging and destruction-induced drug release are determined by the size and composition of both imaging and drug-carrying tracers (such flexible, thin-shelled and thick-shelled microbubbles, nanoparticles, and liposomes). The local drug release rate may also be adjusted by the ultrasound energy level.

It should be clear that other similar embodiments are possible: for instance, the tracer agents are injected first, then the therapy zone is visualized through any of the traditional means such as destruction and destruction/reperfusion etc., the therapy parameters are then fine-tuned to adjust the therapy beam, and then subsequently the drug bearing particles are injected and activated by a subsequent therapy beam.

In yet another embodiment, for enhancing the targeting accuracy and drug release efficiency, a mixture of imaging and drug bearing contrast agents (i.e. Ultrasound Particles and Ultrasound Dosage Forms) is injected at the same time and selectively activated for imaging and drug release.

For selective activation, the two different particles are required to be different from each other in terms of ultrasound responsiveness. This can be explained with reference to the following exemplary embodiment.

Thus, at least two populations of microbubbles are well mixed and simultaneously injected (or infused) into bloodstream: a small amount of flexible microbubbles (e.g., lipid microbubbles) for imaging and a relatively large amount of relatively inflexible microcapsules (e.g., drug-bearing polymer microbubbles with a greater activation threshold) for targeted drug delivery.

In order to estimate the amount of drug (i.e., the number of drug-carrying microcapsules) required in the region of interest, a small amount of flexible microbubbles, in conjunction with continuous low-MI contrast imaging, is used to estimate and determine the contrast amount over a period of time by evaluating the concentration and contrast kinetics (such as wash-in, wash-out, as well as fractional blood volume) of the flexible microbubbles at a region of interest. In the meantime, a relatively large amount of drug-carrying microcapsules (with a greater activation threshold) arrive in the target region and, at the initiation of the treating clinician, the microcapsules can be opened to release their pharmaceutical payload through the use of ultrasound at a higher intensity (with both greater amplitude and pulse length) above the activation threshold. The activation can be initiated manually by the treating clinician or automatically using a programmable trigger.

Polymer microbubbles with different shell thickness (but similar diameters) or different shell thickness-to-diameter ratios can also be employed as the different populations of microbubbles. This is because polymer microcapsules (possessing the same shell material) with thicker shells are usually harder to be activated (destroyed). The thin-shelled agents are either already echogenic at low MI's or ideally destroyed (intermittently over a given time period) for wash-in/wash-out perfusion kinetic measurements without destruction of the thicker shelled drug bearing agents. This allows multiple assessment or even "high MI" imaging where high MI refers to an MI large enough to destroy the thin shelled bubbles with minimal effect on the thick shelled bubbles. Therefore, by carefully designing the size and shell thickness of the various populations of bubbles, it is possible to design specific ultrasonic activation pressures for specific microbubble populations.

Either the thin-shell imaging agent or a third population of ultrasound contrast microbubbles (e.g., medium-thick, polymer shelled, gas-filled microcapsules with an acoustic destruction greater than that for the thin-shell imaging bubble but less than that for thick-shelled, drug-bearing microcapsules) can be used for the visualization of the focus of therapeutic ultrasound beam. The third population serves to enhance the surrounding tissue permeability.

In the case of only the thin-shelled imaging agent, the limited bubbles destruction or radiation force-induced bubble motion around the focal region of each therapeutic ultrasound beam can be utilized for the visualization of the therapeutic beams. In the case of both thin and medium-thick shelled agents, the visualization can be realized by transmitting two ultrasound pulses and compare their respective echoes: the first pulse with a lower amplitude disrupts the thin-shelled imaging agent only while the second pulse with a slightly greater amplitude destroys both the thin- and medium-thick shelled and the gas microcapsules. It is further possible to calibrate the required therapeutic ultrasound intensity across the treatment zone using a medium-thick contrast agent with a well defined destruction threshold: the ultrasound intensity can be adjusted at different depths so that the agent destruction starts to occur.

The minimum and maximum ultrasound energy levels required for non-destructive contrast imaging and destruction-induced drug release are determined by the size and composition of thin, medium thick, and thick-shelled microbubbles.

The microbubble used for detection of the presence of bubbles reacts reversibly to the applied ultrasound at low mechanical index and the drug-filled microcapsule does not react at low MI but gives a signal only at high MI. This requires a lipid-shelled, or alternatively a thin albumin-shelled bubble as the imaging bubble and a hard-shelled bubble as the ultrasonically-activatable drug delivery vehicle.

A thick-shelled microcapsule has a very low scattering capability (i.e., scattering cross-section) and thus "invisible" (especially in harmonic imaging modes) before the microcapsule is acoustically activated (i.e., disrupted or disintegrated) at low acoustic pressures usually used in the low MI contrast imaging. Therefore, the presence of thick-shelled microcapsules introduces insignificant attenuation and causes little reduction in imaging sensitivity for the low-MI contrast imaging with the thin-shelled microbubbles.

The ratio between the thin-shelled imaging microbubbles and the thick shelled drug delivery agent is chosen such that sufficient drug can be incorporated, therefore the ratio can be 1:10 or even 1:100 or more. For drug delivery applications it is not always necessary to limit the intensities to the imaging range. So a higher pressure required to open the thick-shelled microcapsules does not have to pose a problem.

Ideally, microbubble and microcapsule populations used for contrast imaging and drug release have the same surface characteristics (but different shell thickness). In this embodiment the drug-carrying agent is a hard-shelled microcapsule such as polymer-shelled microbubbles, this implies that in order to make the same surface characteristics, the imaging agent may also be a polymer bubble. The advantage here is that the biodistribution of the imaging bubble is exactly the same as for the drug-carrying bubble thereby allowing use of the imaging bubble to infer the quantity and presence of the less echogenic drug release bubbles.

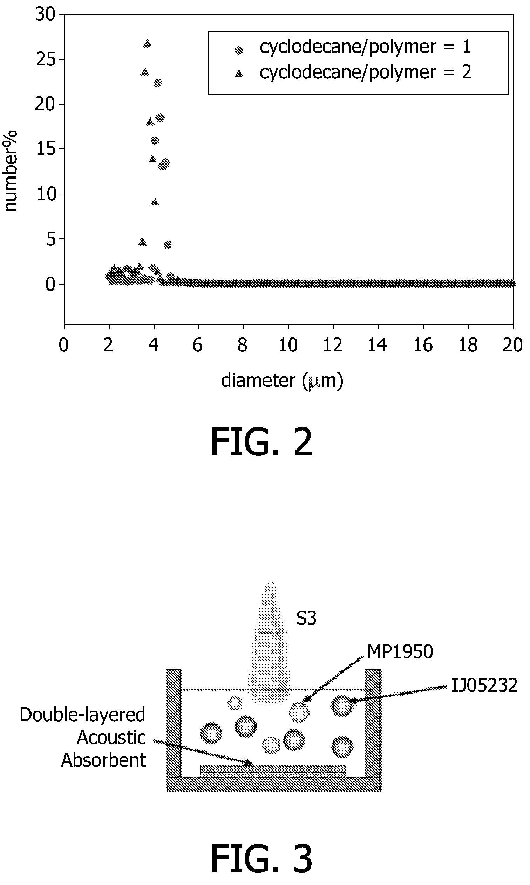

The invention can be realized with reference to the existing manufacture of contrast agents with well-defined size distributions (narrow or mono-dispersed) with repeatable and robust shell characteristics (chemical makeup, thickness). The size distribution of two polymer microcapsules samples is presented in Fig. 2. This careful control over the size of the microbubbles and the shell characteristics ensures that there is a sharp pressure transition point where the microbubble moves from a completely encapsulated particle to an empty shell with available drug or genetic material. An additional advantage of this technology is that the microbubbles all contain a known quantity of drug (a simple number density times the drug per particle), which make quantification of the delivery of pharmaceutical intervention simpler.

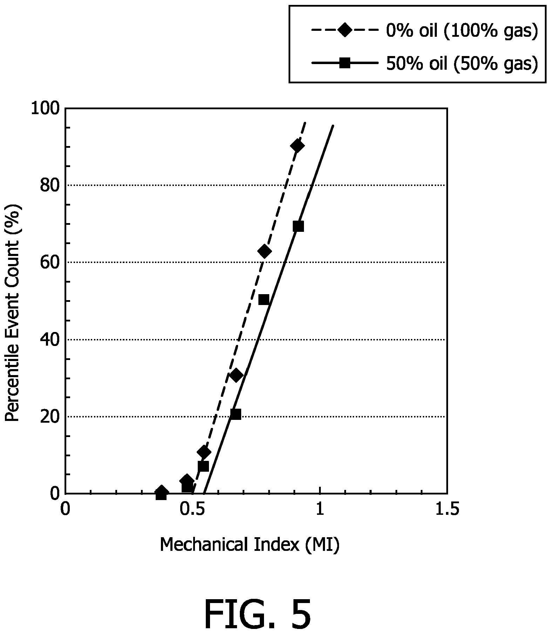

Therapeutic Agents such as drugs are preferably incorporated in an additional oil phase, creating partially oil-filled, partially gas filled capsules. An example of a drug that can be present in such an oil phase is paclitaxel or other anti-cancer agents that can be used at low concentrations. Having polymer-shelled tracer capsules that are gas filled and therapeutic polymer shelled capsules that are partially filled with oil containing a therapeutic a preferred embodiment can be realized. The size distribution of the capsules is not affected if the same preparation method (inkjet printing or premix emulsification) is used for synthesizing partially oil filled or completely gas filled capsules. However, the acoustic threshold is influenced if capsules are partially oil filled as demonstrated in Fig. 5.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

### BRIEF DESCRIPTION OF THE DRAWINGS

The invention will be illustrated with reference to the following, non-limiting Example and the accompanying non-limiting Figures.
Fig. 1 depicts a flow chart for illustration of the planning and treatment phases in the aforementioned two-phase process.\
Fig. 2 shows the size distributions of two inkjetted samples of microcapsules.
Fig. 3 provides a schematic description of the experimental set up discussed in the Example. An imaging contrast agent MP1950 and a polymer-shelled microcapsule agent IJ05232 (as the drug vehicle) are first mixed and then injected into deionized water. IJ05232 is a polymer-based thin-shelled microcapsule agent (polymer/core=4; 90nm shell thickness with a low but sharp threshold of 0.25 in MI). The description of the preparation of these microcapsules can be found in Bohmer et al. Colloids and Surfaces A, 2006 (289) 96-104 for monodisperse polymer shelled capsules. Reference is also made to WO 2006/003581 A1.

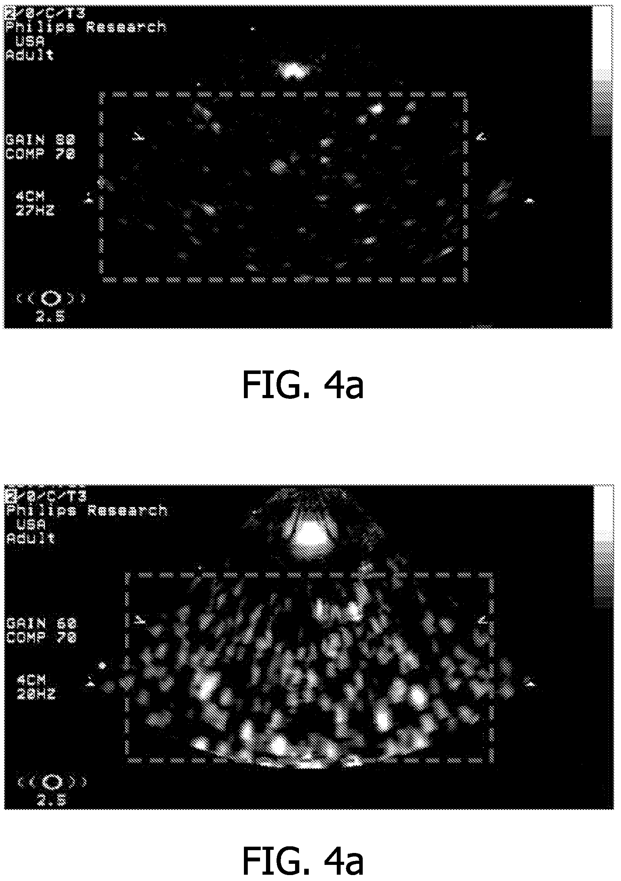

Fig. 4 depicts two consecutive image frames: (a) in the Monitor Mode with a very low transmit power of -22 dB (corresponding to a "derated" MI=0.1 on display), only the imaging agent MP1950 microbubbles were seen with a high receive gain of 80 dB; and (b) in the Impulse Mode with a greater transmit power of -12 dB (corresponding to a "derated" MI=0.4 on display), both the imaging agent MP1950 microbubbles and the polymer agent IJ05232 microcapsules were observed with a lower receive gain of 60 dB as they were disrupted by the intense ultrasound in the Impulse Mode.
Fig. 5 shows the percentile event count as a function of Mechanical Index of 1 MHz, 32 cycle insonation pulses for both hollow and oil-enclosed microcapsule samples. Each microcapsule activation is counted as one event. A sharp activation threshold for air-filled microcapsule is around an MI of 0.5 according to the linear fitting (the blue dashed line) for the measured percentile event counts above 4%). The threshold for activation of oil-enclosed microcapsules is increased to 0.55 according to the linear fitting (the green solid line).

### DETAILED DESCRIPTION OF THE EMBODIMENTS

As demonstrated in Fig. 3, a mixture of a lipid-based imaging agent (MP1950, University of Virginia) and a polymer-based thick-shelled microcapsule agent (IJ05232 with a diameter of 3.26 µm, Philips Research) were first mixed and then injected into a water tank. A sector ultrasound probe S3 connected to a Sonos 7500 ultrasound scanner was positioned vertically and a piece of double-layered acoustic absorbent material placed on the bottom. The ultrasound scanner was operated using a contrast modality consisting of a low-MI Monitor Mode for continuous contrast imaging and an Impulse Mode (with a much higher MI) for the contrast agent destruction. As shown in Fig. 4a in the Monitor Mode with a very low transmit power of -22 dB (corresponding to a "derated" MI=0.1 on display or actual MI ∼0.16 at 3cm in water), only the imaging agent MP1950 microbubbles were seen even with a high receive gain of 80 dB. In Fig. 4b in the Impulse Mode with a greater transmit power of-12 dB (corresponding to a "derated" MI=0.4 on display or actual MI ∼0.52 at 3cm in water), both the imaging agent MP1950 microbubbles and the polymer agent IJ05232 microcapsules were observed with a lower receive gain of 60 dB as they both were disrupted by the intense ultrasound in the Impulse Mode. The receive gain was lowered to compensate the increase in the transmit power.

This approach has the advantage that the imaging bubble can be interrogated repeatedly and its specific signature can be detected. In the case of targeted bubbles both the imaging bubble and the drug delivery vehicle can be modified with the same targeting moiety or in a separate embodiment, the therapeutic agents may not be targeted but in flow next to the targeted imaging microbubbles.

## Claims

1. A composition comprising a first population of ultrasound particles comprising a therapeutic agent, **characterised in that** said composition further comprises a second population of ultrasound particles without therapeutic agent, for use in a method wherein the composition is administered and subjected to ultrasound, the second population of ultrasound particles serving as imaging tracers by being subjected to detection of acoustic properties, the first population of ultrasound particles serving as delivery vehicles comprising and releasing the therapeutic agent.

2. The composition according to claim 1, wherein the second population comprises particles that are echogenic even at low ultrasound intensities and wherein the first population comprises particles that are minimally echogenic below an activation ultrasound threshold.

3. The composition according to claim 1 or 2, wherein the second population comprises particles with a relatively low activation threshold and wherein the first population comprises particles with a relatively high activation threshold.

4. The composition according to any one of the claims 1 to 3, wherein the second population comprises relatively thin-shelled Ultrasound Particles and the second population comprises relatively thick-shelled Ultrasound Particles with the same shell composition and structure.

5. The composition according to claims 1 to 4 wherein said second ultrasound particles gas filled microspheres.

6. The composition according to claim 5, wherein The first population is contained in particles that are activated by heat delivered with focused ultrasonic waves.

## Patentansprüche

1. Zusammensetzung umfassend eine erste Population von ultraschallaktivierten Partikeln, die einen therapeutischen Wirkstoff umfassen, **dadurch gekennzeichnet, dass** die genannte Zusammensetzung weiterhin eine zweite Population von ultraschallaktivierten Partikeln ohne therapeutischen Wirkstoff umfasst, zur Verwendung in einem Verfahren, in dem die Zusammensetzung verabreicht wird und Ultraschall unterzogen wird, wobei die zweite Population von ultraschallaktivierten Partikeln als Bildgebungs-Tracer dient, die einer Detektion von akustischen Eigenschaften unterzogen werden, während die erste Population von ultraschallaktivierten Partikeln als Zuführungsvehikel dient, die den therapeutischen Wirkstoff umfassen und freisetzen.

2. Zusammensetzung nach Anspruch 1, wobei die zweite Population Partikel umfasst, die auch bei geringen Ultraschallintensitäten echogen sind, und wobei die erste Population Partikel umfasst, die unter einem Aktivierungsultraschallschwellwenwert minimal echogen sind.

3. Zusammensetzung nach Anspruch 1 oder 2, wobei die zweite Population Partikel mit einem relativ niedrigen Aktivierungsschwellenwert umfasst und wobei die erste Population Partikel mit einem relativ hohen Aktivierungsschwellenwert umfasst.

4. Zusammensetzung nach einem der Ansprüche 1 bis 3, wobei die zweite Population relativ dünnschalige ultraschallaktivierte Partikel umfasst und die erste Population relativ dickschalige ultraschallaktivierte Partikel mit der gleichen Schalenzusammensetzung und Struktur umfasst.

5. Zusammensetzung nach den Ansprüchen 1 bis 4, wobei die genannten zweiten ultraschallaktivierten Partikel gasgefüllte Mikrosphären sind.

6. Zusammensetzung nach Anspruch 5, wobei die erste Population in Partikeln enthalten ist, die durch Wärme aktiviert werden, welche mit fokussierten Ultraschallwellen zugeführt wird.

## Revendications

1. Composition comprenant une première population de particules à ultrasons comprenant un agent thérapeutique, **caractérisée en ce que** ladite composition comprend en outre une deuxième population de particules à ultrasons sans agent thérapeutique, à utiliser dans un procédé dans lequel la composition est administrée et soumise à des ultrasons, la deuxième population de particules à ultrasons servant de traceurs d'imagerie en étant soumises à une détection de propriétés acoustiques, la première population de particules à ultrasons servant de véhicules de distribution comprenant et libérant l'agent thérapeutique.

2. Composition selon la revendication 1, dans laquelle la deuxième population comprend des particules qui sont échogènes même à des intensités d'ultrasons basses et dans laquelle la première population comprend des particules qui sont minimalement échogènes sous un seuil d'ultrasons d'activation.

3. Composition selon la revendication 1 ou 2, dans laquelle la deuxième population comprend des particules ayant un seuil d'activation relativement bas et dans laquelle la première population comprend des particules ayant un seuil d'activation relativement élevé.

4. Composition selon l'une quelconque des revendications 1 à 3, dans laquelle la deuxième population comprend des particules à ultrasons à enveloppe relativement mince et la première population comprend des particules à ultrasons à enveloppe relativement épaisse avec la même composition et la même structure d'enveloppe.

5. Composition selon les revendications 1 à 4 dans laquelle lesdites deuxièmes particules à ultrasons sont des microsphères remplies de gaz.

6. Composition selon la revendication 5, dans laquelle la première population est contenue dans des particules qui sont activées par la chaleur distribuée avec des ondes ultrasonores concentrées.
